# EUROPEAN PATENT SPECIFICATION

(11) **EP 1 490 496 B1**
(45) Date of publication and mention of the grant of the patent: **23.12.2009**
(21) Application number: 03714146.2
(22) Date of filing: 13.03.2003
(51) Int. Cl.: C12N 15/82, A01H 5/00, A01H 5/10, C07H 21/04

(54) **EARLY INFLORESCENCE-PREFERRED REGULATORY ELEMENTS AND USES THEREOF**
BEIM FRÜHEN BLÜTENSTAND BEVORZUGTE REGULATIONSELEMENTE UND VERWENDUNGEN DAVON
ELEMENTS DE REGULATION FAVORABLES A L'INFLORESCENCE PRECOCE ET LEURS UTILISATIONS

(30) Priority: 13.03.2002 US 364065 P
(43) Date of publication of application: 29.12.2004
(73) Proprietor: PIONEER HI-BRED INTERNATIONAL, INC., Des Moines, Iowa 50309 (US)
(72) Inventor: NUI, Xiping, Pioneer Hi-Bred International, Johnston, IA 50131 (US); BATE, Nicholas, Pioneer Hi-Bred International, Johnston, IA 50131 (US)
(74) Representative: Bentham, Andrew
(86) International application number: PCT/US2003/007861
(87) International publication number: WO 2003/078590

(56) References cited:
- WO-A-00/23578
- US-A- 6 002 069
- US-A- 6 025 483
- US-B1- 6 355 863
- KRIZEK B.A. ET AL.: "use of APETALA1 promoter to assay the in vivo function of chimeric MADS box genes" SEX PLANT REPROD, vol. 12, 1999, pages 14-26, XP002349607
- CHEN, L., ET AL.: "EMF genes regulate Arabidopsis inflorescence development" THE PLANT CELL, vol. 9, 1997, pages 2011-2024, XP002349608
- GOCAL G.F.W. ET AL: 'Evolution of floral meristem identity genes. Analysis of Lolium temulentum genes related to APETALA1 and LEAFY of arabidopsis' PLANT PHYSIOLOGY vol. 125, April 2001, pages 1788 - 1801, XP002979028

### SEQUENCE LISTING

<110> PIONEER HI-BRED INTERNATIONAL, INC.
<120> Early Inflorescence Preferred Regulatory Elements and Uses Thereof
<130> PHI1489
<160> 2
<170> PatentIn version 3.0
<210> 1
   <211> 2197
   <212> DNA
   <213> Zea mays
<400> 1
   cctttttctt tttctccaca acatgaacct tactagaaca ctgccccact taaaagaatg    60
   agggtagaac tcttgaatct tagggatttg aactccttgc agtacctcat aacaagggtg  120
   ttacatgtcc ttcttctgct gttgctgctt gagcaggata tagagagatg accgacaccg   180
   ggttgatctt gggacaacct tcttctcatc ttttcttcgt tgttttcttt tctattctca   240
   ctaccttttt ctttctcttt gttcttccca ctggaggatt ctatcaaaaa gtattaccat   300
   catacagagg aggaacccga agactatgaa ccatgtacaa cagtcttcaa cccaagaatc   360
   accaagcatt gtgatcttag gggcgaggga gtggaaaatg gagttgcttg tgatttggca   420
   gagggaattt tatcaggagt gttttgcttt gagtggaatg ggaactgagg gagttgttgg   480
   gggggggggg tttataggcg agtgggagtg ctcgggtgcg gagtgtggtg atggaacagg   540
   tgacatgagg tagcaggtcg atggaggggg gctgttgccg gcgatgatgg cggcggtggg   600
   tgcgctgcaa aggagggcgt ggggcggtgg tagtgcgcat ggaggcgggc acgcgtgcgg   660
   ggggcacaag tgagtggtgg ggtcgatgac cctgatgttt gtggtctctg gttccaagaa   720
   tctttgtctc tctttatgat aataacttct tttgtcgtcc ttttctgttt actttgactc   780
   aggggcagtg ctttgattct cacggtcggt ccttttgact gagtgactgg acatgtttct   840
   tctgtagcat tgtacaacat gtactttgtg caagctacaa ggccacattt tttgaagcat   900
   agattctttc ccccaaacaa tttatacaaa tatgcaaggc tacacttctt gtatttctat   960
   aacattgtac attcatgaca gaggctcaaa agcttgtaaa ttttgtgcag gtttaattca 1020
   tgtaaagttc ccttgtagag tcatgacaac atcgtactat aaaattattc tacaaaaacc 1080
   acacatgacc cccatgttat ttggtgacaa tacagaaacc acacatctag tgatgatata 1140
   acactgtaca gaagccacaa attataatat ataaaacact atacaaagta tccaaataaa 1200
   gcctaatagg tatggagggt aacctgaatc tttcctaata ataatgaata atctacaata 1260
   atgatttgtt tggacaaaga gaattaaacg gtattgagtg ggctaaaatt ccttgttatt 1320
   caaaaccctc aatcacagtt tctccgaggg aaaaagaaac aggggaggac actcaggctg 1380
   ttcacaatag ggatttcata tcgctctttc caacaatgcc acatcatcaa aagtgttatg 1440
   aaactaaaaa tgaaataata cttctcaatg caaactttca ttttcataga ttaatatact 1500
   aattaaatga tgcaactaaa taaccaatag atgttagtaa aatatggtaa gattaaacaa 1560
   accactatca atggacattt cacatagttt ccaagacttt gaaaacgggt tgacatgatt 1620
   tcatccacat caaactaatt ttatctctga aacccattca ttttaaatga tatggcataa 1680
   cgtccaaaat gctgacgtga cataccatta aatgtgcatg aaactcccat aaaactttta 1740
   ttgataatag cctcacagac atccggtcct acacccgtgt ggacccatca gccagacgcc 1800
   ctgcagcaaa cgcgacgttt gacttgccat ctcgctccct tgtgcccgac cgaccctgga 1860
   aggctggact ggaactggaa caagcaaaat ggaaaaaacc atatctcacc actgaaccgc 1920
   acccttccgg cccacgccag gctcgaccaa tccctgcccc gcgcgccctg acgagcgcat 1980
   cactcgaacg ccggcctcgc taggcccatc cttctggccc gcaataacga tccccgtcat 2040
   gatccgacgg tctagctgcc tccacgccgc tccaaaaccc ccgcgtccaa tcaaaacacg 2100
   acagcgggac gagcgaaacc accgtggttt cgccaaaccg ctttccttcc catctaaaac 2160
   cgccccctcc cttcctcttc tcctagctct cttgcct 2197
<210> 2
   <211> 6183
   <212> DNA
   <213> synthetic
<400> 2
   agcgcccaat acgcaaaccg cctctccccg cgcgttggcc gattcattaa tgcagctggc 60
   acgacaggtt tcccgactgg aaagcgggca gtgagcgcaa cgcaattaat gtgagttagc 120
   tcactcatta ggcaccccag gctttacact ttatgcttcc ggctcgtatg ttgtgtggaa 180
   ttgtgagcgg ataacaattt cacacaggaa acagctatga ccatgattac gccaagcttg 240
   gtaccgagct cggatccact agtaacggcc gccagtgtgc tggaattcgc ccttactata 300
   gggcacgcgt ggtcgacggc ccgggctggt cctttttctt tttctccaca acatgaacct 360
   tactagaaca ctgccccact taaaagaatg agggtagaac tcttgaatct tagggatttg 420
   aactccttgc agtacctcat aacaagggtg ttacatgtcc ttcttctgct gttgctgctt 480
   gagcaggata tagagagatg accgacaccg ggttgatctt gggacaacct tcttctcatc 540
   ttttcttcgt tgttttcttt tctattctca ctaccttttt ctttctcttt gttcttccca 600
   ctggaggatt ctatcaaaaa gtattaccat catacagagg aggaacccga agactatgaa 660
   ccatgtacaa cagtcttcaa cccaagaatc accaagcatt gtgatcttag gggcgaggga 720
   gtggaaaatg gagttgcttg tgatttggca gagggaattt tatcaggagt gttttgcttt 780
   gagtggaatg ggaactgagg gagttgttgg gggggggggg tttataggcg agtgggagtg 840
   ctcgggtgcg gagtgtggtg atggaacagg tgacatgagg tagcaggtcg atggaggggg 900
   gctgttgccg gcgatgatgg cggcggtggg tgcgctgcaa aggagggcgt ggggcggtgg 960
   tagtgcgcat ggaggcgggc acgcgtgcgg ggggcacaag tgagtggtgg ggtcgatgac 1020
   cctgatgttt gtggtctctg gttccaagaa tctttgtctc tctttatgat aataacttct 1080
   tttgtcgtcc ttttctgttt actttgactc aggggcagtg ctttgattct cacggtcggt 1140
   ccttttgact gagtgactgg acatgtttct tctgtagcat tgtacaacat gtactttgtg 1200
   caagctacaa ggccacattt tttgaagcat agattctttc ccccaaacaa tttatacaaa 1260
   tatgcaaggc tacacttctt gtatttctat aacattgtac attcatgaca gaggctcaaa 1320
   agcttgtaaa ttttgtgcag gtttaattca tgtaaagttc ccttgtagag tcatgacaac 1380
   atcgtactat aaaattattc tacaaaaacc acacatgacc cccatgttat ttggtgacaa 1440
   tacagaaacc acacatctag tgatgatata acactgtaca gaagccacaa attataatat 1500
   ataaaacact atacaaagta tccaaataaa gcctaatagg tatggagggt aacctgaatc 1560
   tttcctaata ataatgaata atctacaata atgatttgtt tggacaaaga gaattaaacg 1620
   gtattgagtg ggctaaaatt ccttgttatt caaaaccctc aatcacagtt tctccgaggg 1680
   aaaaagaaac aggggaggac actcaggctg ttcacaatag ggatttcata tcgctctttc 1740
   caacaatgcc acatcatcaa aagtgttatg aaactaaaaa tgaaataata cttctcaatg 1800
   caaactttca ttttcataga ttaatatact aattaaatga tgcaactaaa taaccaatag 1860
   atgttagtaa aatatggtaa gattaaacaa accactatca atggacattt cacatagttt 1920
   ccaagacttt gaaaacgggt tgacatgatt tcatccacat caaactaatt ttatctctga 1980
   aacccattca ttttaaatga tatggcataa cgtccaaaat gctgacgtga cataccatta 2040
   aatgtgcatg aaactcccat aaaactttta ttgataatag cctcacagac atccggtcct 2100
   acacccgtgt ggacccatca gccagacgcc ctgcagcaaa cgcgacgttt gacttgccat 2160
   ctcgctccct tgtgcccgac cgaccctgga aggctggact ggaactggaa caagcaaaat 2220
   ggaaaaaacc atatctcacc actgaaccgc acccttccgg cccacgccag gctcgaccaa 2280
   tccctgcccc gcgcgccctg acgagcgcat cactcgaacg ccggcctcgc taggcccatc 2340
   cttctggccc gcaataacga tccccgtcat gatccgacgg tctagctgcc tccacgccgc 2400
   tccaaaaccc ccgcgtccaa tcaaaacacg acagcgggac gagcgaaacc accgtggttt 2460
   cgccaaaccg ctttccttcc catctaaaac cgccccctcc cttcctcttc tcctagctct 2520
   cttgcctgcg caccactcga gtcgagactc gagaggcacc tgatctttca agggcgaatt 2580
   ctgcagatat ccatcacact ggcggccgct cgagcatgca tctagagggc ccaattcgcc 2640
   ctatagtgag tcgtattaca attcactggc cgtcgtttta caacgtcgtg actgggaaaa 2700
   ccctggcgtt acccaactta atcgccttgc agcacatccc cctttcgcca gctggcgtaa 2760
   tagcgaagag gcccgcaccg atcgcccttc ccaacagttg cgcagcctga atggcgaatg 2820
   ggacgcgccc tgtagcggcg cattaagcgc ggcgggtgtg gtggttacgc gcagcgtgac 2880
   cgctacactt gccagcgccc tagcgcccgc tcctttcgct ttcttccctt cctttctcgc 2940
   cacgttcgcc ggctttcccc gtcaagctct aaatcggggg ctccctttag ggttccgatt 3000
   tagagcttta cggcacctcg accgcaaaaa acttgatttg ggtgatggtt cacgtagtgg 3060
   gccatcgccc tgatagacgg tttttcgccc tttgacgttg gagtccacgt tctttaatag 3120
   tggactcttg ttccaaactg gaacaacact caaccctatc gcggtctatt cttttgattt 3180
   ataagggatt ttgccgattt cggcctattg gttaaaaaat gagctgattt aacaaattca 3240
   gggcgcaagg gctgctaaag gaaccggaac acgtagaaag ccagtccgca gaaacggtgc 3300
   tgaccccgga tgaatgtcag ctactgggct atctggacaa gggaaaacgc aagcgcaaag 3360
   agaaagcagg tagcttgcag tgggcttaca tggcgatagc tagactgggc ggttttatgg 3420
   acagcaagcg aaccggaatt gccagctggg gcgccctctg gtaaggttgg gaagccctgc 3480
   aaagtaaact ggatggcttt cttgccgcca aggatctgat ggcgcagggg atcaagatct 3540
   gatcaagaga caggatgagg atcgtttcgc atgattgaac aagatggatt gcacgcaggt 3600
   tctccggccg cttgggtgga gaggctattc ggctatgact gggcacaaca gacaatcggc 3660
   tgctctgatg ccgccgtgtt ccggctgtca gcgcaggggc gcccggttct ttttgtcaag 3720
   accgacctgt ccggtgccct gaatgaactg caggacgagg cagcgcggct atcgtggctg 3780
   gccacgacgg gcgttccttg cgcagctgtg ctcgacgttg tcactgaagc gggaagggac 3840
   tggctgctat tgggcgaagt gccggggcag gatctcctgt catctcgcct tgctcctgcc 3900
   gagaaagtat ccatcatggc tgatgcaatg cggcggctgc atacgcttga tccggctacc 3960
   tgcccattcg accaccaagc gaaacatcgc atcgagcgag cacgtactcg gatggaagcc 4020
   ggtcttgtcg atcaggatga tctggacgaa gagcatcagg ggctcgcgcc agccgaactg 4080
   ttcgccaggc tcaaggcgcg catgcccgac ggcgaggatc tcgtcgtgat ccatggcgat 4140
   gcctgcttgc cgaatatcat ggtggaaaat ggccgctttt ctggattcaa cgactgtggc 4200
   cggctgggtg tggcggaccg ctatcaggac atagcgttgg atacccgtga tattgctgaa 4260
   gagcttggcg gcgaatgggc tgaccgcttc ctcgtgcttt acggtatcgc cgctcccgat 4320
   tcgcagcgca tcgccttcta tcgccttctt gacgagttct tctgaattga aaaaggaaga 4380
   gtatgagtat tcaacatttc cgtgtcgccc ttattccctt ttttgcggca ttttgccttc 4440
   ctgtttttgc tcacccagaa acgctggtga aagtaaaaga tgctgaagat cagttgggtg 4500
   cacgagtggg ttacatcgaa ctggatctca acagcggtaa gatccttgag agttttcgcc 4560
   ccgaagaacg ttttccaatg atgagcactt ttaaagttct gctatgtcat acactattat 4620
   cccgtattga cgccgggcaa gagcaactcg gtcgccgggc gcggtattct cagaatgact 4680
   tggttgagta ctcaccagtc acagaaaagc atcttacgga tggcatgaca gtaagagaat 4740
   tatgcagtgc tgccataacc atgagtgata acactgcggc caacttactt ctgacaacga 4800
   tcggaggacc gaaggagcta accgcttttt tgcacaacat gggggatcat gtaactcgcc 4860
   ttgatcgttg ggaaccggag ctgaatgaag ccataccaaa cgacgagagt gacaccacga 4920
   tgcctgtagc aatgccaaca acgttgcgca aactattaac tggcgaacta cttactctag 4980
   cttcccggca acaattaata gactggatgg aggcggataa agttgcagga ccacttctgc 5040
   gctcggccct tccggctggc tggtttattg ctgataaatc tggagccggt gagcgtgggt 5100
   ctcgcggtat cattgcagca ctggggccag atggtaagcc ctcccgtatc gtagttatct 5160
   acacgacggg gagtcaggca actatggatg aacgaaatag acagatcgct gagataggtg 5220
   cctcactgat taagcattgg taactgtcag accaagttta ctcatatata ctttagattg 5280
   atttaaaact tcatttttaa tttaaaagga tctaggtgaa gatccttttt gataatctca 5340
   tgaccaaaat cccttaacgt gagttttcgt tccactgagc gtcagacccc gtagaaaaga 5400
   tcaaaggatc ttcttgagat cctttttttc tgcgcgtaat ctgctgcttg caaacaaaaa 5460
   aaccaccgct accagcggtg gtttgtttgc cggatcaaga gctaccaact ctttttccga 5520
   aggtaactgg cttcagcaga gcgcagatac caaatactgt ccttctagtg tagccgtagt 5580
   taggccacca cttcaagaac tctgtagcac cgcctacata cctcgctctg ctaatcctgt 5640
   taccagtggc tgctgccagt ggcgataagt cgtgtcttac cgggttggac tcaagacgat 5700
   agttaccgga taaggcgcag cggtcgggct gaacgggggg ttcgtgcaca cagcccagct 5760
   tggagcgaac gacctacacc gaactgagat acctacagcg tgagcattga gaaagcgcca 5820
   cgcttcccga agggagaaag gcggacaggt atccggtaag cggcagggtc ggaacaggag 5880
   agcgcacgag ggagcttcca gggggaaacg cctggtatct ttatagtcct gtcgggtttc 5940
   gccacctctg acttgagcgt cgatttttgt gatgctcgtc aggggggcgg agcctatgga 6000
   aaaacgccag caacgcggcc tttttacggt tcctggcctt ttgctggcct tttgctcaca 6060
   tgttctttcc tgcgttatcc cctgattctg tggataaccg tattaccgcc tttgagtgag 6120
   ctgataccgc tcgccgcagc cgaacgaccg agcgcagcga gtcagtgagc gaggaagcgg 6180
   aag 6183

## Description

### BACKGROUND OF THE INVENTION

Expression of isolated DNA sequences in a plant host is dependent upon the presence of operably linked regulatory elements that are functional within the plant host. Choice of the regulatory sequences will determine when and where within the organism the isolated DNA sequence is expressed. Where continuous expression is desired throughout the cells of a plant, constitutive promoters are utilized. In contrast, where gene expression in response to a stimulus is desired, inducible promoters are the regulatory element of choice. Where expression in specific tissues or organs are desired, tissue-preferred promoters and/or terminators are used. That is, these regulatory elements can drive expression in specific tissues or organs. Additional regulatory sequences upstream and/or downstream from the core sequences can be included in expression cassettes of transformation vectors to bring about varying levels of expression of isolated nucleotide sequences in a transgenic plant.

Plants have two basic growth modes during their life cycles -- vegetative growth and flower and seed growth. The above ground vegetative growth of the plant develops from the apical meristem. This vegetative meristem gives rise to all of the leaves that are found on the plant. The plant will maintain its vegetative growth pattern until the apical meristem undergoes a change. This change actually alters the identity of the meristem from a vegetative to an inflorescence meristem. The inflorescence meristem produce small leaves before it next produces floral meristems. It is the floral meristem from which the flower develops.

The floral meristem undergoes a series of developmental changes that eventually give rise to the four basic structures of the flower, sepals, petals, stamens and carpels. Each of these structures is derived sequentially from a whorl that develops from the floral meristem. Whorl 1 is the first to appear, and it develops into the sepals of the plant. The second whorl develops into petals. The third and fourth whorls define the stamen (male reproductive organs) and carpel (female reproductive organs), respectively.

From a genetic perspective, two phenotypic changes that control vegetative and floral growth are programmed in the plant. The first genetic change involves the switch from the vegetative to the floral state. If this genetic change is not functioning properly, then flowering will not occur. The second genetic event follows the commitment of the plant to form flowers. The observation that the organs of the plant develop in a sequential manner suggests that a genetic mechanism exists in which a series of genes are sequentially turned on and off. This switching is necessary for each whorl to obtain its final unique identity.

A series of *Arabidopsis* mutants have been identified in which normal flowers are replaced with structures that resemble infloresce meristems and the shoots that normally develop from them. One such mutant is LEAFY. This mutant does not contain any normal flowers. Instead, the early flower structures that develop appear as inflorescence shoots, whereas the later flowers partially resemble normal flowers. These later developing flowers contain sepal and carpel-like structures; that is they lack petals and stamens. This suggests that LEAFY has two functions committing the plant to floral meristem development and defining petals and stamens.

Flowers of APETALA1 mutants are not altered as dramatically as LEAFY mutants. These mutants express a partial inflorescence meristem phenotype where secondary floral meristems appear in the axis region of the sepal. But when the APETALA1 and LEAFY mutants are combined, the flowers appears as an inflorescence shoot. The snapdragon analog to the APETALA1 gene, SQUAMOSA is much more severe, and the flowers appear as inflorescence shoots. APETALA1 also affects the normal development of sepals and petals.

Cloning of the *Arabidopsis* gene involved in the commitment to flowering and the genes controlling flower organ development has been achieved by either heterologous probing with snapdragon genes or by transposon tagging. In general, sequence analysis of these genes suggests they can be classified as a MADS-box containing gene or a gene with another structure.

MADS-box genes play a central role in the development of flowers in plants. The fact that snapdragon MADS-box genes were used as heterologous probes itself is evidence that this class of genes is important in other species. Further evidence of their importance has been demonstrated by the fact that homologous sequences have been found in monocots such as maize.

Maize is a monocotyledonous plant species and belongs to the grass family. It is unusual for a flowering plant as it has unisexual inflorescences The male inflorescence (tassel) develops in a terminal position, whereas the female inflorescences (ears) grow in the axil of vegetative leaves. The inflorescences, as typical for grasses, are composed of spikelets. In the case of maize each spikelet contains two florets (the grass flower) enclosed by a pair of bracts (inner and outer glume).

Each floret consists of two enclosing bracts (lemma and palea), two lodicules (scale-like organs, prominent only in the male flowers), three stamens and a central pistil enclosing a single ovule.

The grass flower is sufficiently different from a typical angiosperm flower. The latter is composed of concentric whorls of sepals and petals enclosing whorls of stamens and pistils. The homologies of the angiosperm flower-tissues to those of the grass floret have been debated for more than 200 years.

According to the invention, developmentally specific regulatory sequences are disclosed which enable the transcription of genes during the critical time of florescence development, such as tissues preferred to the early flowering tissues such as meristems to manipulate flowering time, flower initiation, and meristem development in plants.

Isolation and characterization of early flower development-preferred promoters and terminators that can serve as regulatory elements for expression of isolated nucleotide sequences of interest in a flowering-preferred manner are needed for improving flowering traits in plants.

### SUMMARY OF THE INVENTION

According to the invention there is provided herein a regulatory element isolated from Maize which comprises the following: a TATA box and is capable of driving expression similar to that of the AP1-like gene in plant cells, particularly maize plant cells. The promoter is known as ZM-MADS PRO1, also as ZAP(Zea mays APETALA) but shall hereinafter be referred to as AP-1like. The invention also comprises expression constructs comprising the regulatory elements of the invention operably linked to DNA sequences, vectors incorporating said expression constructs, plant cells transformed with these constructs and resultant plants regenerated from the same. The regulatory elements of the invention provide for expression of operably linked sequences in tissues involved in flowering in maize.

### DETAILED DESCRIPTION OF THE INVENTION

The AP1 gene regulates specific stages of flowering development in a range of species. In accordance with the invention, nucleotide sequences are provided that allow initiation of transcription in tissues involved in early flowering development such as meristem tissue, in an AP1-like expression pattern. The sequences of the invention comprise transcriptional initiation regions associated temporally with flower development and spatially with flower development tissues. Thus, the compositions of the present invention comprise novel nucleotide sequences for plant regulatory elements natively associated with the nucleotide sequences coding for maize AP1-like gene.

A method for expressing an isolated nucleotide sequence in a plant using the transcriptional initiation sequences disclosed herein is provided. The method comprises transforming a plant cell with a transformation vector that comprises an isolated nucleotide sequence operably linked to one or more of the plant regulatory sequences of the present invention and regenerating a stably transformed plant from the transformed plant cell. In this manner, the regulatory sequences are useful for controlling the expression of endogenous as well as exogenous products in a flower development-preferred manner.

Under the transcriptional initiation regulation of the flowering development-specific region will be a sequence of interest, which will provide for modification of the phenotype of the developing flower. Such modification includes modulating the production of an endogenous product, as to amount, relative distribution, or the like, or production of an exogenous expression product to provide for a novel function or product in the flower, manipulating the size of the flower, manipulating the time of flowering, length of flowering and the like.

By "flowering development" is intended favored expression in the newly developing florescence tissues including but not limited to meristem tissue.

By "regulatory element" is intended sequences responsible for tissue and temporal expression of the associated coding sequence including promoters, terminators, enhancers, introns, and the like.

By "terminator" is intended sequences that are needed for termination of transcription. A regulatory region of DNA that causes RNA polymerase to disassociate from DNA, causing termination of transcription.

By "promoter" is intended a regulatory region of DNA usually comprising a TATA box capable of directing RNA polymerase II to initiate RNA synthesis at the appropriate transcription initiation site for a particular coding sequence. A promoter can additionally comprise other recognition sequences generally positioned upstream or 5' to the TATA box, referred to as upstream promoter elements, which influence the transcription initiation rate. It is recognized that having identified the nucleotide sequences for the promoter region disclosed herein, it is within the state of the art to isolate and identify further regulatory elements in the 5' untranslated region upstream from the particular promoter region identified herein. Thus the promoter region disclosed herein is generally further defined by comprising upstream regulatory elements such as those responsible for tissue and temporal expression of the coding sequence, enhancers and the like. In the same manner, the promoter elements which enable expression in the desired tissue such as the ear can be identified, isolated, and used with other core promoters to confirm early ear and flowering development-preferred expression.

The isolated promoter sequences of the present invention can be modified to provide for a range of expression levels of the isolated nucleotide sequence. Less than the entire promoter region can be utilized and the ability to drive flowering development-preferred expression retained. However, it is recognized that expression levels of mRNA can be decreased with deletions of portions of the promoter sequence. Thus, the promoter can be modified to be a weak or strong promoter. Generally, by "weak promoter" is intended a promoter that drives expression of a coding sequence at a low level. By "low level" is intended levels of about 1/10,000 transcripts to about 1/100,000 transcripts to about 1/500,000 transcripts. Conversely, a strong promoter drives expression of a coding sequence at a high level, or at about 1/10 transcripts to about 1/100 transcripts to about 1/1,000 transcripts. Generally, at least about 20 nucleotides of an isolated promoter sequence will be used to drive expression of a nucleotide sequence.

It is recognized that to increase transcription levels enhancers can be utilized in combination with the promoter regions of the invention. Enhancers are nucleotide sequences that act to increase the expression of a promoter region. Enhancers are known in the art and include the SV40 enhancer region, the 35S enhancer element, and the like.

The promoters of the present invention can be isolated from the 5' untranslated region flanking its respective transcription initiation site. Likewise the terminator can be isolated from the 3' untranslated region flanking its respective stop codon. The term "isolated" refers to material, such as a nucleic acid or protein, which is: (1) substantially or essentially free from components which normally accompany or interact with the material as found in its naturally occurring environment or (2) if the material is in its natural environment, the material has been altered by deliberate human intervention to a composition and/or placed at a locus in a cell other than the locus native to the material. Methods for isolation of promoter regions are well known in the art.

The sequences for the promoter region is set forth in SEQ ID NO: 1. The Apl-like promoter set forth in SEQ ID NO: 1 is 2197 nucleotides in length.

The promoter regions of the invention may be isolated from any plant, including, but not limited to corn *(Zea mays),* canola *(Brassica napus, Brassica rapa ssp.),* alfalfa *(Medicago sativa),* rice *(Oryza sativa),* rye *(Secale cereale),* sorghum *(Sorghum bicolor, Sorghum vulgare),* sunflower *(Helianthus annuus),* wheat *(Triticum aestivum),* soybean *(Glycine* max), tobacco *(Nicotiana tabacum),* potato *(Solanum tuberosum),* peanuts *(Arachis hypogaea),* cotton *(Gossypium hirsutum),* sweet potato *(Ipomoea batatus),* cassava *(Manihot esculenta),* coffee *(Cofea spp.),* coconut *(Cocos nucifera),* pineapple *(Ananas comosus),* citrus trees *(Citrus spp.),* cocoa *(Theobroma cacao),* tea *(Camellia sinensis),* banana *(Musa spp.),* avocado *(Persea americana),* fig *(Ficus casica),* guava *(Psidium guajava),* mango *(Mangifera indica),* olive *(Olea europaea),* oats, barley, vegetables, ornamentals, and conifers. Preferably, plants include com, soybean, sunflower, safflower, canola, wheat, barley, rye, alfalfa, and sorghum.

Promoter sequences from other plants may be isolated according to well-known techniques based on their sequence homology to the promoter sequences set forth herein. In these techniques, all or part of the known promoter sequence is used as a probe which selectively hybridizes to other sequences present in a population of cloned genomic DNA fragments (i.e. genomic libraries) from a chosen organism. Methods are readily available in the art for the hybridization of nucleic acid sequences.

The entire promoter sequence or portions thereof can be used as a probe capable of specifically hybridizing to corresponding promoter sequences. To achieve specific hybridization under a variety of conditions, such probes include sequences that are unique and are preferably at least about 10 nucleotides in length, and most preferably at least about 20 nucleotides in length. Such probes can be used to amplify corresponding promoter sequences from a chosen organism by the well-known process of polymerase chain reaction (PCR). This technique can be used to isolate additional promoter sequences from a desired organism or as a diagnostic assay to determine the presence of the promoter sequence in an organism. Examples include hybridization screening of plated DNA libraries (either plaques or colonies; see e.g. Innis et al. (1990) PCR Protocols, A Guide to Methods and Applications, eds., Academic Press).

The terms "stringent conditions" or "stringent hybridization conditions" includes reference to conditions under which a probe will hybridize to its target sequence, to a detectably greater degree than other sequences (e.g., at least 2-fold over background). Stringent conditions are target-sequence dependent and will differ depending on the structure of the polynucleotide. By controlling the stringency of the hybridization and/or washing conditions, target sequences can be identified which are 100% complementary to a probe (homologous probing). Alternatively, stringency conditions can be adjusted to allow some mismatching in sequences so that lower degrees of similarity are detected (heterologous probing). Generally, probes of this type are in a range of about 250 nucleotides in length to about 1000 nucleotides in length.

An extensive guide to the hybridization of nucleic acids is found in Tijssen, Laboratory Techniques in Biochemistry and Molecular Biology-Hybridization with Nucleic Acid Probes, Part I, Chapter 2 "Overview of principles of hybridization and the strategy of nucleic acid probe assays", Elsevier, New York (1993); and Current. Protocols in Molecular Biology, Chapter 2, Ausubel, et al., Eds., Greene Publishing and Wiley-Interscience, New York (1995). See also Sambrook et al. (1989) Molecular Cloning: A Laboratory Manual (2nd ed. Cold Spring Harbor Laboratory, Cold Spring Harbor, N.Y.).

In general, sequences that correspond to the promoter sequence of the present invention and hybridize to the promoter sequence disclosed herein will be at least 50% homologous, 55% homologous, 60% homologous, 65% homologous, 70% homologous, 75% homologous, 80% homologous, 85% homologous, 90% homologous, 95% homologous and even 98% homologous or more with the disclosed sequence.

Specificity is typically the function of post-hybridization washes, the critical factors being the ionic strength and temperature of the final wash solution. Generally, stringent wash temperature conditions are selected to be about 5°C to about 2°C lower than the melting point (Tₘ) for the specific sequence at a defined ionic strength and pH. The melting point, or denaturation, of DNA occurs over a narrow temperature range and represents the disruption of the double helix into its complementary single strands. The process is described by the temperature of the midpoint of transition, Tₘ, which is also called the melting temperature. Formulas are available in the art for the determination of melting temperatures.

Hybridization conditions for the promoter sequences of the invention include hybridization at 42°C in 50%(w/v) formamide, 6X SSC, 0.5%(w/v) SDS, 100mg/ml salmon sperm DNA. Exemplary low stringency washing conditions include hybridization at 42°C in a solution of 2X SSC, 0.5% (w/v) SDS for 30 minutes and repeating. Exemplary moderate stringency conditions include a wash in 2X SSC, 0.5% (w/v) SDS at 50°C for 30 minutes and repeating. Exemplary high stringency conditions include a wash in 2X SSC, 0.5% (w/v) SDS, at 65°C for 30 minutes and repeating. Sequences that correspond to the promoter of the present invention may be obtained using all the above conditions.

The following terms are used to describe the sequence relationships between two or more nucleic acids or polynucleotides: (a) "reference sequence", (b) "comparison window", (c) "percentage of sequence identity", and (d) "substantial identity".
(a) As used herein, "reference sequence" is a defined sequence used as a basis for sequence comparison. A reference sequence may be a subset or the entirety of a specified sequence; for example, as a segment of a full-length promoter sequence, or the complete promoter sequence.
(b) As used herein, "comparison window" makes reference to a contiguous and specified segment of a polynucleotide sequence, wherein the polynucleotide sequence may be compared to a reference sequence and wherein the portion of the polynucleotide sequence in the comparison window may comprise additions or deletions (i.e., gaps) compared to the reference sequence (which does not comprise additions or deletions) for optimal alignment of the two sequences. Generally, the comparison window is at least 20 contiguous nucleotides in length and optionally can be 30, 40, 50, 100, or more contiguous nucleotides in length. Those of shill in the art understand that to avoid a high similarity to a reference sequence due to inclusion of gaps in the polynucleotide sequence a gap penalty is typically introduced and is subtracted from the number of matches.
(c) As used herein, "percentage of sequence identity" means the value determined by comparing two optimally aligned sequences over a comparison window, wherein the portion of the polynucleotide sequence in the comparison window may comprise additions or deletions (i.e., gaps) as compared to the reference sequence (which does not comprise additions or deletions) for optimal alignment of the two sequences. The percentage is calculated by determining the number of positions at which the identical nucleic acid base occurs in both sequences to yield the number of matched positions, dividing the number of matched positions by the total number of positions in the window of comparison and multiplying the result by 100 to yield the percentage of sequence identity.
(d) The term "substantial identity" of polynucleotide sequences means that a polynucleotide comprises a sequence that has at least 70% sequence identity, preferably at least 80%, more preferably at least 90% and most preferably at least 95%, compared to a reference sequence using one of the alignment programs described using standard parameters.

Methods of aligning sequences for comparison are well known in the art. Gene comparisons can be determined by conducting BLAST (Basic Local Alignment Search Tool; Altschul, S. F., et al., (1993) J. Mol. Biol. 215:403-410; see also www.ncbi.nlm.nih.gov/BLAST/) searches under default parameters for identity to sequences contained in the BLAST "GENEMBL" database. A sequence can be analyzed for identity to all publicly available DNA sequences contained in the GENEMBL database using the BLASTN algorithm under the default parameters. Identity to the sequence of the present invention would mean a polynucleotide sequence having at least 65% sequence identity, more preferably at least 70% sequence identity, more preferably at least 75% sequence identity, more preferably at least 80% identity, more preferably at least 85% sequence identity, more preferably at least 90% sequence identity and most preferably at least 95% sequence identity wherein the percent sequence identity is based on the entire promoter region.

GAP uses the algorithm of Needleman and Wunsch (J. Mol. Biol. 48:443-453, 1970) to find the alignment of two complete sequences that maximizes the number of matches and minimizes the number of gaps. GAP considers all possible alignments and gap positions and creates the alignment with the largest number of matched bases and the fewest gaps. It allows for the provision of a gap creation penalty and a gap extension penalty in units of matched bases. GAP must make a profit of gap creation penalty number of matches for each gap it inserts. If a gap extension penalty greater than zero is chosen, GAP must, in addition, make a profit for each gap inserted of the length of the gap times the gap extension penalty. Default gap creation penalty values and gap extension penalty values in Version 10 of the Wisconsin Genetics Software Package for protein sequences are S and 2, respectively. For nucleotide sequences the default gap creation penalty is 50 while the default gap extension penalty is 3. The gap creation and gap extension penalties can be expressed as an integer selected from the group of integers consisting of from 0 to 200. Thus, for example, the gap creation and gap extension penalties can be 0, 1, 2, 3, 4, 5, 6, 7, 8, 9, 10,15, 20, 25, 30,35,40, 45, 50, 55,60,65 or greater.

GAP presents one member of the family of best alignments. There may be many members of this family, but no other member has a better quality. GAP displays four figures of merit for alignments: Quality, Ratio, Identity, and Similarity. The Quality is the metric maximized in order to align the sequences. Ratio is the quality divided by the number of bases in the shorter segment. Percent Identity is the percent of the symbols that actually match. Percent Similarity is the percent of the symbols that are similar. Symbols that are across from gaps are ignored. A similarity is scored when the scoring matrix value for a pair of symbols is greater than or equal to 0.50, the similarity threshold. The scoring matrix used in Version 10 of the Wisconsin Genetics Software Package is BLOSUM62 (see Henikoff & Henikoff (1989) Proc. Natl. Acad. Sci. USA 89:10915).

Sequence fragments with high percent identity to the sequences of the present invention also refer to those fragments of a particular regulatory element nucleotide sequence disclosed herein that operate to promote the immature ear (early flowering)-preferred expression of an operably linked isolated nucleotide sequence. These fragments will comprise at least about 100 contiguous nucleotides of the particular promoter nucleotide sequence disclosed herein. The nucleotides of such fragments will usually comprise the TATA recognition sequence of the particular promoter sequence. Such fragments can be obtained by use of restriction enzymes to cleave the naturally occurring regulatory element nucleotide sequences disclosed herein; by synthesizing a nucleotide sequence from the naturally occurring DNA sequence; or can be obtained through the use of PCR technology. See particularly, Mullis et al. (1987) Methods Enzymol. 155:335-350, and Erlich, ed. (1989) PCR Technology (Stockton Press, New York). Again, variants of these fragments, such as those resulting from site-directed mutagenesis, are encompassed by the compositions of the present invention.

Nucleotide sequences comprising at least about 100 contiguous sequences of the sequence set forth in SEQ ID NOS:1 or 2 are encompassed These sequences can be isolated by hybridization, PCR, and the like. Such sequences encompass fragments capable of driving immature ear (early flowering)-preferred expression, fragments useful as probes to identify similar sequences, as well as elements responsible for temporal or tissue specificity.

Biologically active variants of the regulatory sequences are also encompassed by the compositions of the present invention. A regulatory "variant" is a modified form of a regulatory sequence wherein one or more bases have been modified, removed or added. For example, a routine way to remove part of a DNA sequence is to use an exonuclease in combination with DNA amplification to produce unidirectional nested deletions of double stranded DNA clones. A commercial kit for this purpose is sold under the trade name Exo-Size™ (New England Biolabs, Beverly, Mass.). Briefly, this procedure entails incubating exonuclease III with DNA to progressively remove nucleotides in the 3' to 5' direction at 5' overhangs, blunt ends or nicks in the DNA template. However, exonuclease III is unable to remove nucleotides at 3', 4-base overhangs. Timed digests of a clone with this enzyme produces unidirectional nested deletions.

One example of a regulatory sequence variant is a promoter formed by one or more deletions from a larger promoter. The 5' portion of a promoter up to the TATA box near the transcription start site can be deleted without abolishing promoter activity, as described by Zhu et al., The Plant Cell 7: 1681-89 (1995). Such variants should retain promoter activity, particularly the ability to drive expression in immature ear (early flowering) or other such tissues. Biologically active variants include, for example, the native regulatory sequences of the invention having one or more nucleotide substitutions, deletions or insertions. Activity can be measured by Northern blot analysis, reporter activity measurements when using transcriptional fusions, and the like. See, for example, Sambrook et al. (1989) Molecular Cloning: A Laboratory Manual (2nd ed. Cold Spring Harbor Laboratory, Cold Spring Harbor, N.Y.).

The nucleotide sequences for the immature ear (early flowering)-preferred regulatory elements disclosed in the present invention, as well as variants and fragments thereof, are useful in the genetic manipulation of any plant when operably linked with an isolated nucleotide sequence whose expression is to be controlled to achieve a desired phenotypic response. By "operably linked" is intended the transcription or translation of the isolated nucleotide sequence is under the influence of the regulatory sequence. In this manner, the nucleotide sequences for the regulatory elements of the invention may be provided in expression cassettes along with isolated nucleotide sequences for expression in the plant of interest, more particularly in the immature ear (early flowering) of the plant. Such an expression cassette is provided with a plurality of restriction sites for insertion of the nucleotide sequence to be under the transcriptional control of the regulatory elements.

The genes of interest expressed by the regulatory elements of the invention can be used for varying the phenotype of ears and flowering development. This can be achieved by increasing expression of endogenous or exogenous products in developing corn ears. Alternatively, the results can be achieved by providing for a reduction of expression of one or more endogenous products, particularly enzymes or cofactors in the developing corn ear. These modifications result in a change in phenotype of the transformed plant. It is recognized that the regulatory elements may be used with their native coding sequences to increase or decrease expression resulting in a change in phenotype in the transformed plant.

In another embodiment, the regulatory elements of the invention can be used for callus-preferred expression of selectable markers. For example, regulatory elements such as the Lec1 promoter and terminator would allow plants to be regenerated that have no field resistance to herbicide but may be completely susceptible to the herbicide in the callus stage.

General categories of genes of interest for the purposes of the present invention include for example, those genes involved in information, such as Zinc fingers; those involved in communication, such as kinases; and those involved in housekeeping, such as heat shock proteins. More specific categories of transgenes include genes encoding important traits for agronomics, insect resistance, disease resistance, herbicide resistance, and grain characteristics. Still other categories of transgenes include genes for inducing expression of exogenous products such as enzymes, cofactors, and hormones from plants and other eukaryotes as well as prokaryotic organisms. It is recognized that any gene of interest, including the native coding sequence, can be operably linked to the regulatory elements of the invention and expressed in the developing corn ear tissue.

Exogenous products include plant enzymes and products as well as those from other sources including prokaryotes and other eukaryotes. Such products include enzymes, cofactors, hormones, and the like.

The nucleotide sequence operably linked to the regulatory elements disclosed herein can be an antisense sequence for a targeted gene. By "antisense DNA nucleotide sequence" is intended a sequence that is in inverse orientation to the 5'-to-3' normal orientation of that nucleotide sequence. When delivered into a plant cell, expression of the antisense DNA sequence prevents nominal expression of the DNA nucleotide sequence for the targeted gene. The antisense nucleotide sequence encodes an RNA transcript that is complementary to and capable of hybridizing with the endogenous messenger RNA (mRNA) produced by transcription of the DNA nucleotide sequence for the targeted gene. In this case, production of the native protein encoded by the targeted gene is inhibited to achieve a desired phenotypic response. Thus the regulatory sequences disclosed herein can be operably linked to antisense DNA sequences to reduce or inhibit expression of a native protein in the plant ear or to regulate the development if the ear or flower.

The expression cassette will also include at the 3' terminus of the isolated nucleotide sequence of interest, a transcriptional and translational termination region functional in plants. The termination region can be native with the promoter nucleotide sequence of the present invention, can be native with the DNA sequence of interest, or can be derived from another source.

Convenient termination regions are available from the Ti-plasmid of A. *tumefaciens,* such as the octopine synthase and nopaline synthase termination regions. See also: Guerineau et al. (1991) Mol. Gen. Genet. 262:141-144; Proudfoot (1991) Cell 64:671-674; Sanfacon et al. (1991) Genes Dev. 5:141-149; Mogen et al. (1990) Plant Cell 2:1261-1272; Munroe et al. (1990) Gene 91:151-158; Ballas et al. 1989) Nucleic Acids Res. 17:7891-7903; Joshi et al. (1987) Nucleic Acid Res. 15:9627-9639.

The expression cassettes can additionally contain 5' leader sequences. Such leader sequences can act to enhance translation. Translation leaders are known in the art and include: picornavirus leaders, for example: EMCV leader (Encephalomyocarditis 5' noncoding region), Elroy-Stein et al. (1989) Proc. Nat. Acad. Sci. USA 86:6126-6130; potyvirus leaders, for example, TEV leader (Tobacco Etch Virus), Allison et al. (1986); MDMV leader (Maize Dwarf Mosaic Virus), Virology 154:9-20; human immunoglobulin heavy-chain binding protein (BiP), Macejak et al. (1991) Nature 353:90-94; untranslated leader from the coat protein mRNA of alfalfa mosaic virus (AMV RNA 4), Jobling et al. (1987) Nature 325:622-625); tobacco mosaic virus leader (TMV), Gallie et al. (1989) Molecular Biology of RNA, pages 237-256; and maize chlorotic mottle virus leader (MCMV) Lommel et al. (1991) Virology 81:382-385. See also Della-Cioppa et al. (1987) Plant Physiology 84:965-968. The cassette can also contain sequences that enhance translation and/or mRNA stability such as introns.

In those instances where it is desirable to have the expressed product of the isolated nucleotide sequence directed to a particular organelle, particularly the plastid, amyloplast, or to the endoplasmic reticulum, or secreted at the cell's surface or extracellularly, the expression cassette can further comprise a coding sequence for a transit peptide. Such transit peptides are well known in the art and include, but are not limited to: the transit peptide for the acyl canier protein, the small subunit of RUBISCO, plant EPSP synthase, and the like.

In preparing the expression cassette, the various DNA fragments can be manipulated, so as to provide for the DNA sequences in the proper orientation and, as appropriate, in the proper reading frame. Toward this end, adapters or linkers can be employed to join the DNA fragments or other manipulations can be involved to provide for convenient restriction sites, removal of superfluous DNA, removal of restriction sites, or the like. For this purpose, *in vitro* mutagenesis, primer repair, restriction digests, annealing, and resubstitutions such as transitions and transversions, can be involved.

As noted herein, the present invention provides vectors capable of expressing genes of interest under the control of the regulatory elements. In general, the vectors should be functional in plant cells. At times, it may be preferable to have vectors that are functional in *E. coli* (e.g., production of protein for raising antibodies, DNA sequence analysis, construction of inserts, obtaining quantities of nucleic acids). Vectors and procedures for cloning and expression in *E. coli* are discussed in Sambrook *et al.* (supra).

The transformation vector comprising the regulatory sequences of the present invention operably linked to an isolated nucleotide sequence in an expression cassette, can also contain at least one additional nucleotide sequence for a gene to be cotransformed into the organism. Alternatively, the additional sequence(s) can be provided on another transformation vector.

Vectors that are functional in plants can be binary plasmids derived from *Agrobacterium.* Such vectors are capable of transforming plant cells. These vectors contain left and right border sequences that are required for integration into the host (plant) chromosome. At minimum, between these border sequences is the gene to be expressed under control of the regulatory elements of the present invention. In one embodiment, a selectable marker and a reporter gene are also included. For ease of obtaining sufficient quantities of vector, a bacterial origin that allows replication in *E. coli* can be used.

Reporter genes can be included in the transformation vectors. Examples of suitable reporter genes known in the art can be found in, for example: Jefferson et al. (1991) in Plant Molecular Biology Manual, ed. Gelvin et al. (Kluwer Academic Publishers), pp. 1-33; DeWet et al. (1957) Mol. Cell. Biol. 7:725-737; Goff et al. (1990) EMBO J. 9:2517-2522; Kain et al. (1995) BioTechniques 19:650-655; and Chiu et al. (1996) Current Biology 6:325-330.

Selectable marker genes for selection of transformed cells or tissues can be included in the transformation vectors. These can include genes that confer antibiotic resistance or resistance to herbicides. Examples of suitable selectable marker genes include, but are not limited to: genes encoding resistance to chloramphenicol, Herrera Estrella et al. (1983) EMBO J. 2:987-992; methotrexate, Herrera Estrella et al. (1983) Nature 303:209-213; Meijer et al. (1991) Plant Mol. Biol. 16:807-820; hygromycin, Waldron et. al. (1985) Plant Mol. Biol. 5:103-108; Zhijian et al. (1995) Plant Science 108:219-227; streptomycin, Jones et al. (1987) Mol. Gen. Genet. 210:86-91; spectinomycin, Bretagne-Sagnard et al. (1996) Transgenic Res. 5:131-137; bleomycin, Hille et al. (1990) Plant Mol. Biol. 7:171-176; sulfonamide, Guerineau et al. (1990) Plant Mol. Biol. 15:127-136; bromoxynil, Stalker et al. (1988) Science 242:419-423; glyphosate, Shaw et al. (1956) Science 233:478-481; phosphinothricin, DeBlock et al. (1957) EMBO J. 6:2513-2518.

Other genes that could serve utility in the recovery of transgenic events but might not be required in the final product would include, but are not limited to: GUS (b-glucoronidase), Jefferson (1987) Plant Mol. Biol. Rep. 5:387); GFP (green florescence protein), Chalfie et al. (1994) Science 263:802; luciferase, Teeri et al. (1989) EMBO J. 8:343; and the maize genes encoding for anthocyanin production, Ludwig et al. (1990) Science 247:449.

The transformation vector comprising the particular regulatory sequences of the present invention, operably linked to an isolated nucleotide sequence of interest in an expression cassette, can be used to transform any plant. In this manner, genetically modified plants, plant cells, plant tissue, seed, and the like can be obtained. Transformation protocols can vary depending on the type of plant or plant cell, i.e., monocot or dicot, targeted for transformation. Suitable methods of transforming plant cells include microinjection, Crossway et al. (1986) Biotechniques 4:320-334; electroporation, Riggs et al. (1986) Proc. Natl. Acad. Sci. USA 83:5602-5606; *Agrobacterium*-mediated transformation, see for example, Townsend et al. U.S. Patent 5,563,055; direct gene transfer, Paszkowski et al. (1984) EMBO J. 3:2717-2722; and ballistic particle acceleration, see for example, Sanford et al. U.S. Patent 4,945,050; Tomes et al. (1995) in Plant Cell, Tissue, and Organ Culture: Fundamental Methods, ed. Gamborg and Phillips (Springer-Verlag, Berlin); and McCabe et al. (1988) Biotechnology 6:923-926. Also see Weissinger et al. (1988) Annual Rev. Genet. 22:421-477; Sanford et al. (1987) Particulate Science and Technology 5:27-37 (onion); Christou et al. (1988) Plant Physiol. 87:671-674 (soybean); McCabe et al. (1988) Bio/Technology 6:923-926 (soybean); Datta et al. (1990) Biotechnology 8:736-740 (rice); Klein et al. (1988) Proc. Natl. Acad. Sci. USA 85:4305-4309 (maize); Klein et al. (1988) Biotechnology 6:559-563 (maize); Klein et al. (1988) Plant Physiol. 91:440-444 (maize); Fromm et al. (1990) Biotechnology 8:833-839; Hooydaas-Van Slogteren et al. (1984) Nature (London) 311:763-764; Bytebier et al. (1987) Proc. Natl. Acad. Sci. USA 84:5345-5349 (Liliaceae); De Wet et al. (1985) in The Experimental Manipulation of Ovule Tissues, ed. G. P. Chapman et al. (Longman, New York), pp. 197-209 (pollen); Kaeppler et al. (1990) Plant Cell Reports 9:415-418; and Kaeppler et al. (1992) Theor. Appl. Genet. 84:560-566 (whisker-mediated transformation); D.Halluin et al. (1992) Plant Cell 4:1495-1505 (electroporation); Li et al. (1993) Plant Cell Reports 12:250-255 and Christou et al. (1995) Annals of Botany 75:407-413 (rice); Osjoda et al. (1996) Nature Biotechnology 14:745-750 (maize via Agrobacterium tumefaciens).

The cells that have been transformed can be grown into plants in accordance with conventional ways. See, for example, McCormick et al. (1986) Plant Cell Reports 5:81-84. These plants can then be grown and pollinated with the same transformed strain or different strains. The resulting hybrid having immature ear (early flowering)-preferred expression of the desired phenotypic characteristic can then be identified. Two or more generations can be grown to ensure that immature ear (early flowering)-preferred expression of the desired phenotypic characteristic is stably maintained and inherited.

The following examples are offered by way of illustration and not by way of limitation.

### Examples

The regulatory regions of the invention were isolated from maize plants and cloned. The genes were selected as a source for a immature ear (early flowering)-preferred regulatory regions based on the spatial expression of their gene products. The method for their isolation is described below.

### Example 1: Isolation of Promoter Sequences using Genome Walker

### Genomic DNA Isolation

Genomic DNA upstream of the coding sequence for maize AP1-like genes was isolated using the Universal GenomeWalker Kit from CLONTECH (Palo Alto, CA).

### Northern Blot

RNA was isolated from shoots of 4-week seedlings using the TriZol method (Invitrogen, Carlsbad, CA). 15 ug total RNA was separated on 1% agarose MOPS- formaldehyde gels and blotted on Hybond-N+ membrane (Amersham). DNA probes were labeled using RediPrimeII kit (Amersham) and hybridized to membrane in ExpressHyb (CLONTECH, Palo Alto, CA) at 65 °C overnight. The membranes were washed twice in 2XSSC, 0.1% SDS at room temperature and twice in 0.1XSSC, 0.1 % SDS at 50 °C. The membranes were autographed to visualize hybridization signals.

### RESULTS

AP1-like Gene Expression was observed by Northern analysis in a wild type plant (W22); and in a W22 plant introgressed with the teosinte 1L chromosome (TIL), a W22 plant introgressed with the teosinte 1L and 3L chromosomes (T1L3L), a Tb/tb1-mum3 heterozygote (het) and a tb1-mum3 homozygote (tb1), all in W22 background.

### Example 2

A plasmid was prepared incorporating the Ap-1 like promoter of the invention. Its sequence is shown in SEQ ID NO: 2.

## Claims

1. An isolated regulatory element that is capable of driving transcription in an immature ear (early flowering)-preferred manner, said regulatory element comprising a nucleotide sequence selected from:
(a) the nucleotide sequence set forth in SEQ ID NO: 1, or bases 331 - 2527 of SEQ ID NO:2;
(b) a sequence that hybridizes to SEQ ID NO: 1, or to bases 331 - 2527 of SEQ ID NO:2 under highly stringent conditions;
(c) a sequence having at least 65% sequence identity to SEQ ID NO: 1, or to bases 331 - 2527 of SEQ ID NO:2, wherein the % sequence identity is based on the entire sequence and is determined by GAP version 10 analysis using default parameters; and
(d) a sequence comprising at least about 100 nucleotides of the sequence of SEQ ID NO:1 or 2.

2. An expression cassette comprising a regulatory element of claim 1 operably linked to a further nucleotide sequence.

3. An expression cassette of claim 2 wherein said further sequence is a gene.

4. An expression cassette of claim 2, wherein said further nucleotide sequence is an antisense sequence for a targeted gene.

5. An expression cassette of claim 2 or 3, further wherein said further nucleotide sequence encodes a gene product involved in a fatty acid synthesis or a gene product having enhanced amino acid content.

6. A transformation vector comprising an expression cassette of any one of claims 2 to 5.

7. A plant stably transformed with an expression cassette of any one of 2 to 5.

8. A plant of claim 7, which is a monocot.

9. The plant of claim 8, which is a maize, wheat, rice, barley, sorghum, or rye plant.

10. Seed of the plant of any one of claims 7 to 9, which comprises an expression cassette to any one of claims 2 to 5

11. A method for selectively expressing a nucleotide sequence in a plant immature ear comprising transforming a plant cell with a transformation vector of claim 6.

12. A method of claim 11 wherein the regulatory element is capable of initiating transient expression of said further nucleotide sequence in callus tissue.

13. A method of claim 12 further comprising regenerating a stably transformed plant from said transformed plant cell; wherein expression of said further nucleotide sequence alters the phenotype of said plant immature ear.

14. A plant cell stably transformed with an expression cassette of any one of claims 2 to 5.

15. A plant cell of claim 14, wherein said plant cell is from a monocot.

16. A plant cell of claim 15, wherein said plant cell is from maize, wheat, rice, barley, sorghum, or rye.

## Patentansprüche

1. Isoliertes regulatorisches Element, das zum Steuern der Transkription auf eine unreife Ähre bzw. einen unreifen Kolben (frühblühend) bevorzugte Weise befähigt ist, wobei das regulatorische Element eine Nukleotidsequenz umfasst, ausgewählt aus:
(a) der Nukleotidsequenz, die in SEQ ID NO:1 angegeben ist, oder den Basen 331-2527 von SEQ ID NO:2;
(b) einer Sequenz, die mit SEQ ID NO:1 oder mit den Basen 331-2527 von SEQ ID NO:2 unter hochstringenten Bedingungen hybridisiert;
(c) einer Sequenz mit wenigstens 65% Sequenzidentität zu SEQ ID NO:1 oder zu den Basen 331-2527 von SEQ ID NO:2, wobei die % Sequenzidentität auf der gesamten Sequenz basiert und mittels GAP-Version-10-Analyse unter Verwendung von Default-Parametern bestimmt wird; und
(d) einer Sequenz, die wenigstens etwa 100 Nukleotide der Sequenz von SEQ ID NO:1 oder 2 umfasst.

2. Expressionskassette, die ein regulatorisches Element nach Anspruch 1 umfasst, funktionell verknüpft mit einer weiteren Nukleotidsequenz.

3. Expressionskassette nach Anspruch 2, wobei die weitere Sequenz ein Gen ist.

4. Expressionskassette nach Anspruch 2, wobei die weitere Nukleotidsequenz eine Antisense-Sequenz für ein targetiertes Gen ist.

5. Expressionskassette nach Anspruch 2 oder 3, bei der weiterhin die weitere Nukleotidsequenz für ein Genprodukt, das in eine Fettsäuresynthese involviert ist, oder ein Genprodukt, das erhöhten Aminosäuregehalt aufweist, codiert.

6. Transformationsvektor, der eine Expressionskassette nach einem der Ansprüche 2 bis 5 umfasst.

7. Pflanze, die stabil mit einer Expressionskassette nach einem der Ansprüche 2 bis 5 transformiert ist.

8. Pflanze nach Anspruch 7, die eine Monokotyle ist.

9. Pflanze nach Anspruch 8, die eine Mais-, Weizen-, Reis-, Gersten-, Sorghum- oder Roggenpflanze ist.

10. Samen der Pflanze nach einem der Ansprüche 7 bis 9, der eine Expressionskassette nach einem der Ansprüche 2 bis 5 umfasst.

11. Verfahren zum selektiven Exprimieren einer Nukleotidsequenz in einer unreifen Pflanzenähre bzw. einem unreifen Pflanzenkolben, umfassend das Transformieren einer Pflanzenzelle mit einem Transformationsvektor nach Anspruch 6.

12. Verfahren nach Anspruch 11, wobei das regulatorische Element zum Initiieren einer transienten Expression der weiteren Nukleotidsequenz im Callusgewebe befähigt ist.

13. Verfahren nach Anspruch 12, das des Weiteren das Regenerieren einer stabil transformierten Pflanze ausgehend von der transformierten Pflanzenzelle umfasst, wobei die Expression der weiteren Nukleotidsequenz den Phänotyp der unreifen Pflanzenähre bzw. des unreifen Pflanzenkolbens verändert.

14. Pflanzenzelle, die stabil mit einer Expressionskassette nach einem der Ansprüche 2 bis 5 transformiert ist.

15. Pflanzenzelle nach Anspruch 14, wobei die Pflanzenzelle von einer Monokotylen stammt.

16. Pflanzenzelle nach Anspruch 15, wobei die Pflanzenzelle von Mais, Weizen, Reis, Gerste, Sorghum oder Roggen stammt.

## Revendications

1. Élément régulateur isolé qui est capable de commander la transcription dans un épi immature d'une manière préférée (floraison précoce), ledit élément régulateur comprenant une séquence nucléotidique choisie parmi :
(a) la séquence nucléotidique présentée en SEQ ID NO : 1, ou les bases 331-2527 de SEQ ID NO :2 ;
(b) une séquence qui s'hybride à SEQ ID NO : 1, ou aux bases 331-2527 de SEQ ID NO : 2 dans des conditions de stringence élevée ;
(c) une séquence présentant au moins 65 % d'identité de séquence avec SEQ ID NO : 1, ou avec les bases 331-2527 de SEQ ID NO : 2, dans laquelle le pourcentage d'identité de séquence est basé sur la séquence entière et est déterminé par une analyse GAP version 10 en utilisant les paramètres par défaut ; et
(d) une séquence comprenant au moins environ 100 nucléotides de la séquence de SEQ ID NO : 1 ou 2.

2. Cassette d'expression comprenant un élément régulateur de la revendication 1 lié de manière fonctionnelle à une autre séquence nucléotidique.

3. Cassette d'expression de la revendication 2, dans laquelle ladite autre séquence est un gène.

4. Cassette d'expression de la revendication 2, dans laquelle ladite autre séquence nucléotidique est une séquence antisens pour un gène ciblé.

5. Cassette d'expression de la revendication 2 ou 3, dans laquelle en outre ladite autre séquence nucléotidique code pour un produit génique impliqué dans une synthèse d'acide gras ou un produit génique ayant un contenu amélioré en acides aminés.

6. Vecteur de transformation comprenant une cassette d'expression de l'une quelconque des revendications 2 à 5.

7. Plante transformée de manière stable avec une cassette d'expression de l'une quelconque des revendications 2 à 5.

8. Plante de la revendication 7, qui est une monocotylédone.

9. Plante de la revendication 8, qui est un plant de maïs, de blé, de riz, d'orge, de sorgho, ou de seigle.

10. Graine de la plante de l'une quelconque des revendications 7 à 9, qui comprend une cassette d'expression de l'une quelconque des revendications 2 à 5.

11. Méthode pour exprimer sélectivement une séquence nucléotidique dans un épi immature de plante comprenant la transformation d'une cellule végétale avec un vecteur de transformation de la revendication 6.

12. Méthode de la revendication 11, dans laquelle l'élément régulateur est capable d'initier l'expression transitoire de ladite autre séquence nucléotidique dans un tissu de cal.

13. Méthode de la revendication 12, comprenant en outre la régénération d'une plante transformée de manière stable à partir de ladite cellule végétale transformée ; dans laquelle l'expression de ladite autre séquence nucléotidique modifie le phénotype dudit épi immature de plante.

14. Cellule végétale transformée de manière stable avec une cassette d'expression de l'une quelconque des revendications 2 à 5.

15. Cellule végétale de la revendication 14, dans laquelle ladite cellule végétale provient d'une monocotylédone.

16. Cellule végétale de la revendication 15, dans laquelle ladite cellule végétale provient du maïs, du blé, du riz, de l'orge, du sorgho, ou du seigle.
